# EUROPEAN PATENT APPLICATION

(11) **EP 1 586 270 A2**
(43) Date of publication of application: **19.10.2005**
(21) Application number: 05252405.5
(22) Date of filing: 18.04.2005
(51) Int. Cl.: A61B 5/15

(54) **Cap for a lancet**

(30) Priority: 16.04.2004 US 825899; 25.02.2005 US 66936; 25.02.2005 US 66937
(71) Applicant: LIFESCAN, INC., Milpitas, California 95035 (US)
(72) Inventor: Allen, John J., Mendota Heights Minnesota 55118 (US); Rockow, Steven G., Coon Rapids Minnesota 55433 (US); Menendez, Adolfo, Cottage Grove Minnesota 55016 (US)
(74) Representative: Mercer, Christopher Paul

(57) **Abstract**

A cap, a method and an apparatus for lancing a dermal tissue target site includes providing a dermal tissue lancing device with a housing, a lancet (L) that is moveable with respect to the housing, and a cap (100). The cap includes a cap body with an opening therethrough for the lancet to pass through, a proximal end, and a distal end. The cap also includes an attachment mechanism (124) for tiltably attaching the cap body to the dermal tissue lancing device. The attachment mechanism enables the cap body to be free to tilt relative to the housing of the dermal tissue lancing device when the distal end of the cap body is urged against a dermal tissue target site. The method also includes contacting and engaging the distal end of the cap body with a dermal tissue target site such that the cap body tilts, urging the cap body towards the dermal tissue target site such that the cap body applies essentially uniform pressure against the dermal tissue target site to create a target site bulge, and lancing the target site bulge.

## Description

### CROSS-REFERENCE

This application claims the benefit of U.S. Application No. 11/066,936, filed February 25, 2005, which is incorporated herein by reference in its entirety and to which application we claim priority under 35 USC §120. This application claims the benefit of U.S. Application No. 11/066,937, filed February 25, 2005, which is incorporated herein by reference in its entirety and to which application we claim priority under 35 USC §120. This application is a continuation-in-part application of U.S. Application No. 10/825,899, filed April 16, 2004, which is incorporated herein by reference in its entirety and to which application we claim priority under 35 USC §120.

### BACKGROUND OF THE INVENTION

### 1. Field of the Invention

The present invention relates, in general, to methods employing medical devices and, in particular, to methods for lancing dermal tissue.

### 2. Description of the Related Art

Conventional lancing devices generally have a rigid housing and a lancet that can be armed and launched so as to protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A biological fluid sample (e.g., a whole blood sample) can then be expressed from the penetrated target site for collection and analysis. Conventional lancing devices are described in U.S. Patent No. 5,730,753 to Morita, U.S. Patent No. 6,045,567 to Taylor et al. and U.S. Patent No. 6,071,250 to Douglas et al., each of which is incorporated fully herein by reference.

Lancing devices often include a cap with a distal end that engages the target site during use. Such a cap usually has an aperture (i.e., opening), through which the lancet protrudes during use. When a cap is engaged (i.e., contacted) with a target site, pressure is usually applied to the target site prior to launch of the lancet. This pressure urges the cap against the target site for the purpose of creating a target site bulge within the opening of the cap. The lancet is then launched to penetrate the target site bulge. A biological fluid sample, typically blood, is thereafter expressed from the lanced target site bulge. The expressed biological fluid sample can then, for example, be tested for an analyte such as glucose.

However, conventional caps and their associated methods may not serve to reliably produce an adequate volume of biological fluid sample due to insufficient contact between the cap and the target site and/or non-uniform application of pressure on the target site by the cap. The design of conventional caps can also cause discomfort to a user during the lancing procedure. Furthermore, in order to obtain a sufficient volume of biological fluid sample, additional pressure (such as a pumping or milking action) usually must be applied either manually or mechanically to the target site following lancing. This additional pressure can serve to facilitate expression of an adequate volume of biological fluid sample. Examples of mechanical devices designed for such use are described in co-pending U.S. Patent Application Nos. 10/653,023 (published as U.S. Patent Application Publication No. 2004/0249253 on December 9, 2004), 10/861,749 (published as U.S. Patent Application Publication No. 2004/0249254 on December 9, 2004) and U.S. Patent No. 5,951,493, each of which is fully incorporated herein by reference. Unfortunately, such devices can be expensive to manufacture.

The present invention therefore provides:
A cap for a dermal tissue lancing device, the cap comprising:
   a cap body with an opening therethrough for at least a portion of a lancet to pass through; the cap body having:
      a proximal end; and
      a distal end
      an attachment mechanism for tiltably attaching the cap body to the dermal tissue lancing device, whereby the cap body is free to tilt relative to the dermal tissue lancing device when the distal end of the cap body is urged against a dermal tissue target site.

Preferably the cap body includes a saddle-contoured compression surface.

Preferably the cap body includes a cap member and a retainer and the attachment mechanism attaches the retainer to the dermal tissue lancing device.

Preferably the attachment mechanism includes a compliant member.

Preferably the attachment mechanism includes threaded pins and concentrically arranged springs configured to attach the cap body to the dermal tissue lancing device.

Preferably the cap body is free to tilt relative to the dermal tissue lancing device due to clearance between the threaded pins and the cap body.

Preferably the cumulative spring constant of the concentrically arranged springs is in the range of 0.05 to 0.15 kg/mm.

Preferably each of the springs have a force in the range of 0.5 kg-f to 1.3 kg-f.

Preferably the attachment mechanism is configured such that the cap body is free to tilt to a within a predetermined angle range relative to the dermal tissue lancing device

Preferably the predetermined angle range is the range between zero degrees and twenty-five degrees.

The present invention also provides a method and apparatus for lancing a dermal tissue target site, the method comprising:
providing a dermal tissue lancing device, the dermal tissue lancing device including a housing, a lancet that is moveable with respect to the housing, and a cap, the cap having:
   a cap body with an opening therethrough for at least a portion of a lancet to pass through; the cap body having:
      a proximal end; and
      a distal end;
   an attachment mechanism for tiltably attaching the cap body to the dermal tissue lancing device, whereby the cap body is free to tilt relative to the housing of the dermal tissue lancing device when the distal end of the cap body is urged against a dermal tissue target site;
contacting the distal end of the cap body with a dermal tissue target site such that the distal end engages the dermal tissue target site and the cap body tilts relative to the housing of the dermal tissue lancing device;
urging the cap body towards the dermal tissue target site such that the cap body applies essentially uniform pressure against the dermal tissue target site, thereby creating a target site bulge; and
lancing the target site bulge with the dermal tissue lancing device.

Preferably the urging step includes urging the cap toward the dermal tissue target site such that the target site bulge contacts a skin probe of the dermal tissue lancing device.

Preferably the contacting step is such that the cap body tilts within a range between zero degrees and twenty-five degrees relative to the housing.

Preferably the cap body includes a saddle-contoured compression surface.

Preferably the cap body includes a cap member and a retainer and the attachment mechanism attaches the retainer to the housing of the dermal tissue lancing device.

Preferably the attachment mechanism includes a compliant member.

Preferably the attachment mechanism includes threaded pins and concentrically arranged springs configured to attach the cap body to the dermal tissue lancing device.

Preferably the cap body is free to tilt relative to the housing of the dermal tissue lancing device due to clearance between the threaded pins and the cap body.

Preferably the cumulative spring constant of the concentrically arranged springs is in the range of 0.05 to 0.15 kg/mm.

Preferably each of the springs have a force in the range of 0.5 kg-f to 1.3 kg-f.

Preferably the attachment mechanism is configured such that the cap body is free to tilt to a within a predetermined angle range relative to the housing of the dermal tissue lancing device.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIG. 1 is a simplified perspective view of a cap for use with a dermal tissue lancing device according to an exemplary embodiment of the present invention attached to a component of a dermal tissue lancing device;
FIG. 2 is a simplified cross-sectional view of the cap and component of a dermal tissue lancing device of FIG. 1 along line A-A of FIG. 1;
FIG. 3 is a simplified exploded perspective view of the cap and component of a dermal tissue lancing device of FIG. 1, wherein the dashed lines indicate alignment of various elements;
FIG. 4 is a perspective view of the cap of FIG. 1 illustrating a manner in which the cap can tilt relative to a component of a dermal tissue lancing device;
FIG. 5 is a simplified cross-sectional view of the cap and component of a dermal tissue lancing device of FIG. 4 along line B-B of FIG. 4;
FIG. 6 is a flow diagram illustrating a sequence of steps in a process for lancing a target site according to an exemplary embodiment of the present invention; and
FIGs. 7A through 7D are simplified schematic, cross-sectional views depicting various stages of the process of FIG. 6.

### DETAILED DESCRIPTION OF THE INVENTION

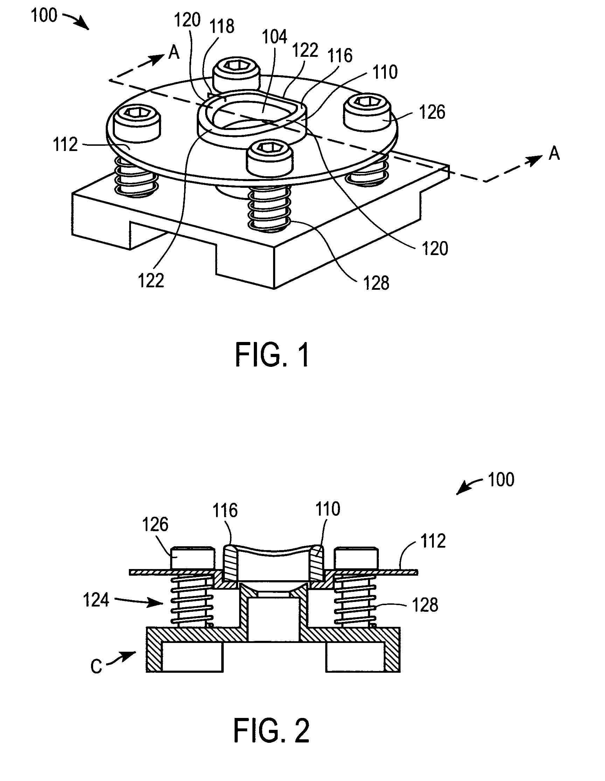

FIGs. 1-3 are various simplified depictions of a cap 100 for use with a dermal tissue lancing device that includes a housing and a lancet moveable with respect to the housing according to an embodiment of the present invention. FIGs. 1-3 depict cap 100 attached to a component (C) of a dermal tissue lancing device. Examples of such components include, but are not limited to, a housing of a dermal tissue lancing device, a skin probe of a dermal tissue lancing device, or other suitable component of a dermal tissue lancing device as is known to one skilled in the art.

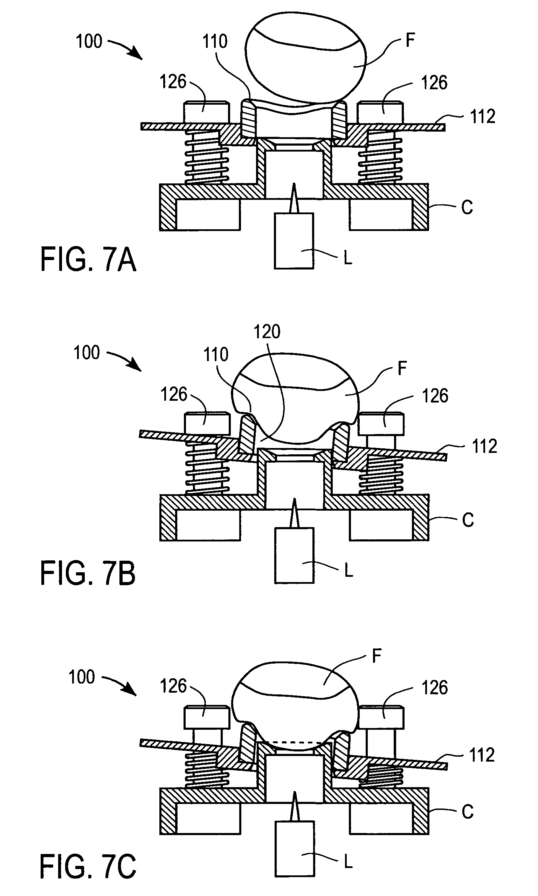

Cap 100 includes a cap body 102 with an opening 104 therethrough for at least a portion of a lancet L (not shown in FIGs. 1-3 but depicted in FIGs. 7A-7D as discussed below) to pass through. Cap body 102 has a proximal end 106 and a distal end 108. In the embodiment of FIGs. 1-3, cap body 102 includes cap member 110 and retainer 112. Furthermore, retainer 112 includes four holes 114 therethrough. However, once apprised of the present disclosure, one skilled in the art will recognize that cap bodies employed in embodiments of the present invention can take any suitable form.

Cap member 110 includes a rim 116 with a saddle-contoured compression surface 118 that forms a continuous ring for engaging a dermal tissue target site when cap 100 is urged toward such a dermal tissue target site. Saddle-contoured compression surface 118 of cap 100 is configured such that opposing first portions 120 of rim 116 are located at a higher elevation than opposing second portions 122 of rim 116 (see, for example, FIG. 1). An example of a such a saddle-contoured compression surface is described in co-pending U.S. Patent Application No. 11/045,542, which is hereby fully incorporated herein by reference. However, any suitable compression surface known to those of skill in the art can be employed in embodiments of caps for dermal lancing devices according to the present invention, including those described in U.S. Patent Application No. 10/706,166, which is fully incorporated herein by reference.

Cap 100 also includes an attachment mechanism 124 for tiltably attaching cap body 102 to component C of the dermal tissue lancing device. As is described in more detail below, attachment mechanism 124 is configured such that cap body 102 can tilt to a predetermined limited degree (i.e. to a predetermined maximum angle) relative to the component of the dermal tissue lancing device when distal end 108 of cap body 102 is urged against a dermal tissue target site. In other words, cap body 102 is free to tilt only within a predetermined angle range relative to the component of the dermal tissue lancing device.

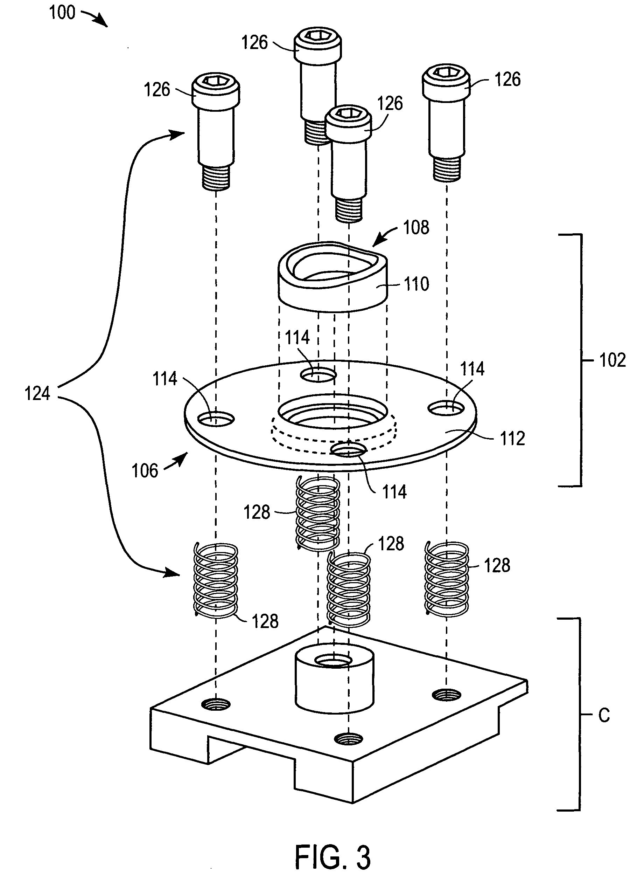

In the embodiment of FIGs. 1-3, attachment mechanism 124 includes four threaded pins 126 and four springs 128, with each of the four springs 128 disposed in a concentric relationship to a different one of the four threaded pins 126 (see, for example, FIG. 3). Springs 128 can be of any suitable force including, for example, springs with a force in the range of 0.5 to 1.3 kg-f. The range of 0.5 kg-f to 1.3 kg-f has been determined to provide for both comfort and the expression of a biological fluid sample. Threaded pins 126 are configured for secure engagement with component C as depicted in FIG. 3.

] Although for the purpose of explanation and illustration only, four sets of threaded pins and springs are depicted in FIGs. 1-3 as included in the attachment mechanism, any suitable number of sets of the threaded pins and springs, sufficient to provide tilting necessary for the invention, can be employed. Moreover, the attachment mechanism of caps according to embodiments of the present invention can take a various forms other than the threaded pins and springs depicted in FIGs. 1-3. For example, the attachment mechanism can be a compliant element configured to tiltably attach a cap body to a component C such as, for example, metal flextures (e.g., leaf springs), elastomeric rods, coil springs, gas springs, pins that are slidably attached to component C (in the vertical direction of FIG. 2) and attached to the cap body via a ball joint or swivel, and combinations thereof.

Springs 128 beneficially serve to provide a relatively equal force along saddle-contoured compression surface 118 of cap 100 when cap 100 is urged against a dermal tissue target site. Ideally, the spring force of each of the four springs 128 would be identical to one another regardless of the amount of compression of each spring 128. However, spring forces increase with compression. Therefore, to minimize any disparity of spring force, it is preferred that springs 128 have a low spring constant. For example, the cumulative spring constant of springs 128 can be, for example, in the range of 0.05 to 0.15 kg/mm.

In the embodiment of FIGs. 1-3, springs 128 also beneficially provide for a target site bulge to be formed prior to component C making contact with the dermal tissue target site (see the discussion of FIGs. 7A through 7D below). This is particularly beneficial when component C is a skin probe.

Once apprised of the present disclosure, one skilled in the art will recognize that a variety of conventional dermal tissue lancing devices can be readily modified for use with caps according to the embodiments of the present invention, including, for example, dermal tissue lancing devices described in the aforementioned U.S. Patent Nos. 5,730,753, 6,045,567 and 6,071,250. Moreover, embodiments of caps according to the present invention can be employed with lancing devices that utilize various techniques for expressing a biological fluid sample from a dermal tissue target site including, but not limited to, techniques that employ lancets, hollow needles, solid needles, micro-needles, ultrasonic extraction devices, or thermal extraction devices. Furthermore, caps according to embodiments of the present invention can be employed with a combined lancing device and integrated meter for testing an analyte (e.g., a meter for testing blood glucose).

Cap 100 comfortably facilitates the flow of a fluid sample (e.g., a blood sample) out of a lanced dermal tissue target site with little or no manipulation (i.e., squeezing and/or milking) of the dermal tissue subsequent to lancing. During use of cap 100, saddle-contoured compression surface 118 is pressed against a target site (e.g., a dermal tissue target site of a user's finger) such that saddle-contoured compression surface 118 engages (i.e., contacts) the dermal tissue target site and creates a target-site bulge within opening 104.

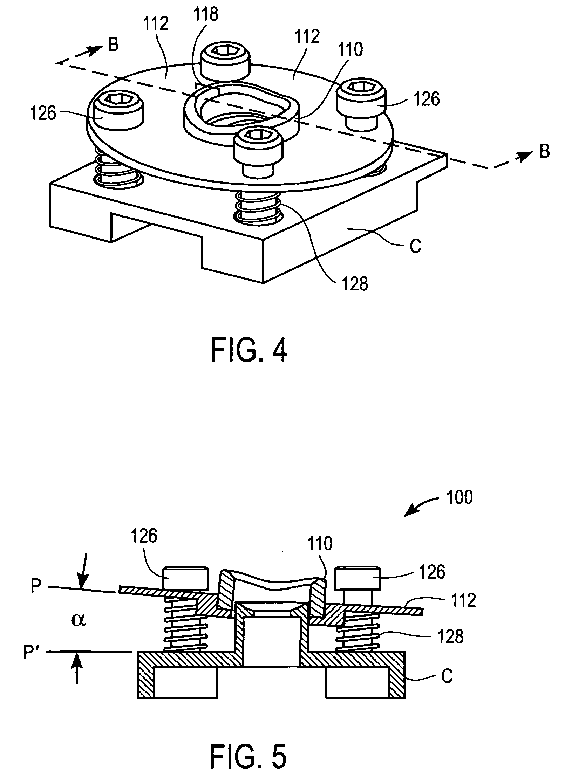

Attachment mechanism 124 beneficially provides limited axial constraint between retainer 112 and component C such that cap body 102 can tilt relative to component C. In this regard, axial constraint refers to the degree to which the longitudinal axis of each hole 114 is compelled to remain parallel with the longitudinal axis of each threaded pin 126. The axial constraint is "limited" in the sense that longitudinal axes of the threaded pins 126 and holes 114 can deviate by a predetermined amount from parallel such that cap body 102 can tilt relative to component C. For example, and referring to FIGs. 4 and 5, cap body 102 can tilt along an axis that is perpendicular to a sectioning plane along line B-B. However, once apprised of the present disclosure, one skilled in the art will recognize that cap body 102 can tilt along various axis other than an axis that is perpendicular to axis B-B.

Such tilting is enabled by a predetermined clearance between threaded pins 126 and holes 114 of retainer 112 and the longitudinal dimension (i.e., length) of holes 114. Furthermore, the degree to which cap body 102 can tilt relative to component C is determined by the dimension of said clearance and said length. For a given clearance, the maximum tilt will decrease as the length of holes 114 increases. The clearance and length dimension of holes 114 can be any suitable dimensions. For example, in the embodiment of FIGs. 1-3, the clearance (i.e., distance between a threaded pin and the retainer when a threaded pin is centered in a hole 114) can be 0.1mm and the length of holes 114 can be 1.0mm. It should also be noted that in the embodiments of FIGs. 1-3, a clearance is provided between component C and cap body 102 within opening 104 in order to avoid unwanted interference between cap body 102 and component C during operation of the lancing device. This clearance can be, for example, in the range of 0.25mm to 0.5mm.

When cap body 102 is tilted relative to component C, a theoretical plane P through retainer 112 forms an angle α with a theoretical plane P' through component C that corresponds to an untilted position of cap body 102 (see FIG. 5). As angle α increases, a component of spring force normal (i.e., perpendicular) to the dermal target site decreases while a component of spring force parallel to the dermal tissue target site increases. During use, the beneficial creation of a target site bulge and expression of a biological fluid sample is driven principally by the normal component of spring force. Therefore, it can be desirable to limit the maximum tilt that can be attained by cap body 102. Angle α (i.e., the predetermined angle of tilt) can be any suitable angle but is typically in the range between 0 to 25 degrees. The tilt enabled by the attachment mechanism provides for a more uniform application of pressure on a dermal tissue target site, by adapting the angle of the cap to the fit the target site. The pressure uniformity improves the expression of a biological fluid sample and improves user comfort..

Cap body 102 can be formed of any suitable material including, for example, a rigid material such as acrylonitrile butadiene styrene plastic, injection moldable plastic, polystyrene and metallic materials or a relatively resiliently deformable material, including, but not limited, to elastomeric materials, polymeric materials, polyurethane materials, latex materials, silicone materials and any combinations thereof.

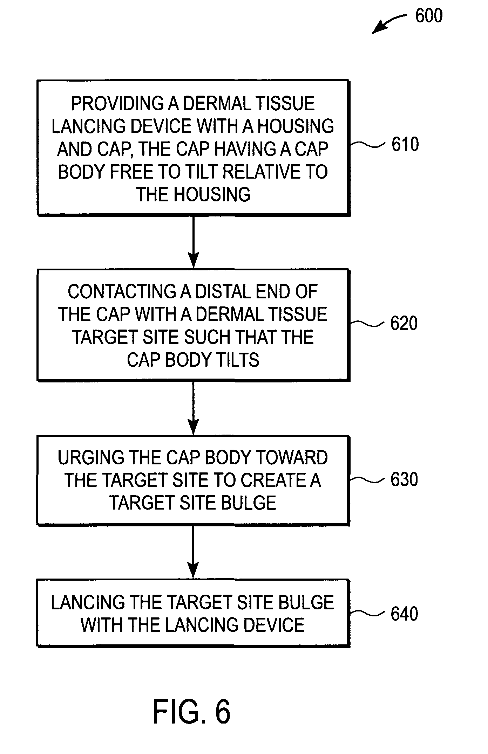

Referring to FIG. 6 and FIGs. 7A through 7D, a process 600 for lancing a dermal tissue target site (e.g., a dermal tissue target site on a user's finger, F) includes providing a dermal tissue lancing device with a housing, a lancet that is moveable with respect to the housing, and a cap (see step 610 of FIG. 6).

The cap of the dermal tissue lancing device includes a cap body with an opening therethrough for at least a portion of the lancet to pass through, a proximal end and a distal end. The cap also includes an attachment mechanism for tiltably attaching (either directly or indirectly) the cap body to the housing of the dermal tissue lancing device, whereby the cap body is free to tilt relative to the housing when the distal end of the cap body is urged against a dermal tissue target site. One skilled in the art will recognize that the cap of process 600 can be, for example, cap 100 of FIGs. 1-5. Therefore, although process 600 can employ any suitable cap, FIGs. 7A through 7D depict cap 100 as described above.

At step 620, the distal end of the cap body is contacted with a dermal tissue target site such that the distal end engages the dermal tissue target site and the cap body tilts relative to the housing of the dermal tissue lancing device (see FIG. 6 and the sequence of FIGs. 7A and 7B). The tilt of the cap body can be, for example, in a range between zero degrees and 25 degrees.

Subsequently, the cap body is urged towards the dermal tissue target site such that the cap body applies essentially uniform pressure against the dermal tissue target site, thereby creating a target site bulge, as set forth in step 630 of FIG. 6. If desired, the cap body can be urged until the target site bulge contacts a component C (e.g., a skin probe) of the dermal tissue lancing device as depicted in FIG. 7C (where a dashed line is employed to indicate an edge of component C hidden behind the target site bulge).

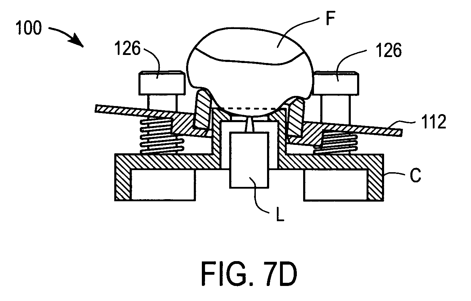

At step 640 of FIG. 6 and as depicted in FIG. 7D (where a dashed line again indicates an edge of component C hidden behind the target site bulge), the target site bulge is lanced with the dermal tissue lancing device.

One embodiment of the present invention, a cap for a dermal tissue lancing device, the cap comprising: a cap body with an opening therethrough for at least a portion of a lancet to pass through; the cap body having: a proximal end; and a distal end an attachment mechanism for tiltably attaching the cap body to the dermal tissue lancing device, whereby the cap body is free to tilt relative to the dermal tissue lancing device when the distal end of the cap body is urged against a dermal tissue target site.

Another embodiment of the present invention, wherein the cap body includes a saddle-contoured compression surface.

Yet another embodiment of the present invention, wherein the cap body includes a cap member and a retainer and the attachment mechanism attaches the retainer to the dermal tissue lancing device.

Yet another embodiment of the present invention, wherein the attachment mechanism includes a compliant member.

Yet another embodiment of the present invention, wherein the attachment mechanism includes threaded pins and concentrically arranged springs configured to attach the cap body to the dermal tissue lancing device.

Yet another embodiment of the present invention, wherein the cap body is free to tilt relative to the dermal tissue lancing device due to clearance between the threaded pins and the cap body.

Yet another embodiment of the present invention, wherein the cumulative spring constant of the concentrically arranged springs is in the range of 0.05 to 0.15 kg/mm.

Yet another embodiment of the present invention, wherein each of the springs have a force in the range of 0.5 kg-f to 1.3 kg-f.

Yet another embodiment of the present invention, wherein the attachment mechanism is configured such that the cap body is free to tilt to a within a predetermined angle range relative to the dermal tissue lancing device.

Yet another embodiment of the present invention, wherein the predetermined angle range is the range between zero degrees and twenty-five degrees.

It should be understood that various alternatives to the embodiments of the invention described herein may be employed in practicing the invention. It is intended that the following claims define the scope of the invention and that caps, methods and apparatuses within the scope of these claims and their equivalents be covered thereby.

## Claims

1. A cap for a dermal tissue lancing device, the cap comprising:
a cap body with an opening therethrough for at least a portion of a lancet to pass through; the cap body having:
a proximal end; and
a distal end
an attachment mechanism for tiltably attaching the cap body to the dermal tissue lancing device, whereby the cap body is free to tilt relative to the dermal tissue lancing device when the distal end of the cap body is urged against a dermal tissue target site.

2. The cap of claim 1, wherein the cap body includes a saddle-contoured compression surface.

3. The cap of claim 1 or claim 2, wherein the cap body includes a cap member and a retainer and the attachment mechanism attaches the retainer to the dermal tissue lancing device.

4. The cap of claim 1, claim 2 or claim 3, wherein the attachment mechanism includes a compliant member.

5. The cap of any one of the preceding claims, wherein the attachment mechanism includes threaded pins and concentrically arranged springs configured to attach the cap body to the dermal tissue lancing device.

6. The cap of claim 5, wherein the cap body is free to tilt relative to the dermal tissue lancing device due to clearance between the threaded pins and the cap body.

7. The cap of claim 5 or claim 6, wherein the cumulative spring constant of the concentrically arranged springs is in the range of 0.05 to 0.15 kg/mm.

8. The cap of claim 5, claim 6 or claim 7, wherein each of the springs have a force in the range of 0.5 kg-f to 1.3 kg-f.

9. The cap of any one of the preceding claims, wherein the attachment mechanism is configured such that the cap body is free to tilt to a within a predetermined angle range relative to the dermal tissue lancing device

10. The cap of claim 9, wherein the predetermined angle range is the range between zero degrees and twenty-five degrees.
